# EUROPEAN PATENT APPLICATION

(11) **EP 3 560 394 A1**
(43) Date of publication of application: **30.10.2019**
(21) Application number: 19170208.3
(22) Date of filing: 18.04.2019
(51) Int. Cl.: A47J 31/00, A47J 27/00

(54) **FERMENTATION DEVICE**

(30) Priority: 23.04.2018 CN 201810366651
(71) Applicant: BSH Hausgeräte GmbH, 81739 München (DE)
(72) Inventor: Xu, Kai, Nanjing, 210005 (CN); ZHU, LINGYAN, Nanjing, 210005 (CN)

(57) **Abstract**

A fermentation device includes: a box, including a sauce-wine pickling area and a curing area; a fermentation chamber, located in the sauce-wine pickling area of the box; and a preserving chamber, located in the curing area of the box, the preserving chamber being isolated from the fermentation chamber. By providing the fermentation chamber and the preserving chamber, isolated from each other, in the fermentation device, different fermentation environments are provided for different flavors of fermented foods, and simultaneous production of different fermented foods is realized, thereby improving the utilization rate of different functions and improving the user experience.

## Description

### BACKGROUND

### Technical Field

The present invention relates to the field of household appliances, and specifically, to a fermentation device.

### Related Art

Fermented foods are mainly produced by utilizing beneficial microorganisms in a natural environment, such as lactic acid bacteria, bacillus aceticus and corynebacterium in yeast, trichobacteria and bacteria, and decomposing organic matters to produce flavor substances after a particular period of fermentation in a specific environment. Chinese traditional foods, such as preserved foods, sauce pickled foods, wine pickled foods and brewed foods are fermented foods. The fermented foods have a unique flavor and are well received by consumers.

In the traditional hand-made craft, to obtain different fermentation environments, different ingredients are selected to be produced in different seasons. For example, sausages are usually produced in December, and filled sausages can be air-dried in a ventilated place for a period of time to complete the production.

With the increasing awareness of food safety, DIY fermented foods have become the choice of a growing number of Chinese families. However, the production of fermented foods by conventional methods requires a user to adjust the specific production time according to the temperature, light and rainfall conditions of the year. This process requires consumers to judge by relying on experience by constantly observing the color and trying the taste.

The use of a fermentation device to produce fermented foods can not only break the seasonal and environmental constraints on production conditions, but also improve the quality of fermented products produced by providing a stable fermentation environment. Therefore, the demand of common families for a domestic fermentation device is increasing.

However, existing domestic fermentation devices often have problems of single function and difficulty in meeting consumer demands.

### SUMMARY

In view of one of the technical problems to be resolved, the present invention provides a fermentation device which can simultaneously produce a plurality of fermented foods, thereby expanding the functions of a domestic fermentation device and improving the user experience.

The present invention provides a fermentation device, including: a box, including a sauce-wine pickling area and a curing area; a fermentation chamber, located in the sauce-wine pickling area of the box; and a preserving chamber, located in the curing area of the box, the preserving chamber being isolated from the fermentation chamber.

The fermentation device includes the fermentation chamber located in the sauce-wine pickling area of the box and the preserving chamber located in the curing area of the box, the preserving chamber being isolated from the fermentation chamber. The preserving chamber and the fermentation chamber, isolated from each other, can provide different fermentation environments for different flavors of fermented foods, so that simultaneous production of different fermented foods is realized. The fermentation device can simultaneously produce at least one type of fermented food at a time, thereby meeting the production needs of home-made fermented foods, effectively improving the utilization rate of different functions of the fermentation device, and effectively improving the user experience of the fermentation device.

Optionally, the volume of the preserving chamber is greater than the volume of the fermentation chamber.

The fermentation chamber is adapted to produce foods with short storage time and high frequency of eating. The preserving chamber is adapted to produce foods with long storage time and a large volume. Therefore, the single-time production of foods produced by the fermentation chamber is relatively small, and the single-time production of foods produced by the preserving chamber is relatively small. Therefore, the volume of the fermentation chamber is small, the volume of the preserving chamber is large, the volume requirements of different fermented foods can be met, and it is beneficial to the improvement of the user experience of the fermentation device.

Optionally, the curing area and the sauce-wine pickling area are arranged in a height direction of the fermentation device.

The arrangement of the sauce-wine pickling area and the curing area in the height direction can effectively improve the installation flexibility of the fermentation device. Especially when the fermentation device is set to be embedded, the method of arranging the sauce-wine pickling area and the curing area in the height direction facilitates the installation and use of embedded device.

Optionally, the curing area is located below the sauce-wine pickling area.

Since the foods produced in the curing area includes meat fermented foods, oil may be separated out from the meat fermented foods during the production process to generate oil droplets. Therefore, the arrangement of the curing area below the sauce-wine pickling area can effectively prevent the oil droplets in the production process of the meat fermented foods from polluting device in the sauce-wine pickling area, thereby effectively avoiding unnecessary pollution during use, and effectively reducing the cleaning difficulty of the fermentation device.

Optionally, the box has an opening. The fermentation device further includes: a door connected to the box, the door being adapted to open or close the opening of the box, and when the door opens the opening of the box, the height of the opening exposed by the door being gradually increased.

The method of opening the door of the fermentation device in the height direction is consistent with the arrangement direction of the sauce-wine pickling area and the curing area, thereby effectively improving the convenience in use of the fermentation device, and also providing greater flexibility for the installation and use of the fermentation device.

Optionally, the door is slidably connected to the box, and the door slides in the height direction of the fermentation device.

Optionally, the door is hingedly connected to the box, and a hinging shaft extends in a width direction of the fermentation device.

The fermentation device is provided with a plurality of doors which is enabled to be opened in the height direction and can be reasonably selected and assembled according to the use requirements. The use convenience can be effectively improved, and it is beneficial to the improvement of the user experience.

Optionally, the fermentation device has a plurality of doors, including: a first door, the first door being adapted to open or close an opening of the sauce-wine pickling area of the box; and a second door, the second door being adapted to open or close an opening of the curing area of the box.

The sauce-wine pickling area of the box and the curing area of the box are provided with different doors, so that the sauce-wine pickling area and the curing area can be opened and closed independently of each other, the isolation between the fermentation chamber and the preserving chamber can be effectively improved, it is beneficial to simultaneous production of different fermented foods, and it is also beneficial to the improvement of the utilization rate of different functions of the fermentation device.

Optionally, the curing area is located below the sauce-wine pickling area, and the second door is a pull-down door.

The method of setting the second door as a pull-down door when the curing area is located below the sauce-wine pickling area can prevent the second door from obstructing the observation of the sauce-wine pickling area, and the use convenience can be effectively improved.

Optionally, there is a plurality of fermentation chambers, isolated from each other.

By providing the plurality of fermentation chambers, which is isolated from each other and separately provides an independent fermentation environment, the simultaneous production of a plurality of fermented foods can be realized. Moreover, the fermentation chamber is adapted to produce foods with short storage time and high frequency of eating. By providing the plurality of fermentation chambers, the sauce-wine pickling area can be divided into a plurality of small spaces, thereby improving the space utilization rate. Moreover, the volume of each fermentation chamber is relatively small, which can reduce the difficulty of controlling fermentation environment parameters such as temperature, humidity and pH value in each fermentation chamber. It is beneficial to the formation of a stable fermentation environment in each fermentation chamber, and is beneficial to the improvement of the quality of fermented foods.

Optionally, the plurality of fermentation chambers includes a first fermentation chamber adapted to perform anaerobic fermentation and a second fermentation chamber adapted to perform aerobic fermentation or anaerobic fermentation. The fermentation device further includes: a first housing member, located in the first fermentation chamber, a space defined by an inner surface of the first housing member being adapted to store materials, and the first housing member being provided with an inlet valve; and a second housing member, located in the second fermentation chamber, a space defined by an inner surface of the second housing member being adapted to store materials, and the second housing member being provided with an inlet valve and an outlet valve.

According to the demands of different fermented foods, fermentation chambers with different fermentation conditions are provided. The function of the fermentation chamber can be effectively expanded, and it is beneficial to the improvement of the utilization rate of different fermentation chambers, to the improvement of the utilization rate of different functions of the fermentation device, and further to the improvement of the user experience of the fermentation device.

Optionally, the volume of the space defined by the inner surface of the second housing member is greater than the volume of the space defined by the inner surface of the first housing member.

Since the second fermentation chamber can perform both aerobic fermentation and anaerobic fermentation and the first fermentation chamber can only perform anaerobic fermentation, the second fermentation chamber has diversified functions. The method of providing a large second fermentation chamber can effectively improve the utilization rate of the second fermentation chamber.

Optionally, a portion of at least one of the first housing member and the second housing member is made of a transparent material.

The method of making a portion of the housing member of a transparent material enables real-time observation of the degree of fermentation of materials in the first housing member and the second housing member, and can better control the degree of fermentation of fermented foods produced in the fermentation chamber. It is beneficial to better achievement of various functions of the fermentation device and to the improvement of the user experience.

Optionally, the device further includes: a bracket, detachably provided in the second housing member, the bracket being adapted to slide in a front-rear direction of the fermentation device to be pushed in or out of the second housing member.

Optionally, the device further includes one or more of a stirrer, a hanger, a tray and a fermentation tank. The stirrer is detachably provided in the fermentation chamber. The hanger is detachably provided in the preserving chamber, the hanger is adapted to slide in the front-rear direction of the fermentation device to be pushed in or out of the preserving chamber, and the hanger is provided with a hook slidable in the hanger. The tray is detachably provided in the preserving chamber, and the tray is adapted to slide in the front-rear direction of the fermentation device to be pushed in or out of the preserving chamber. The fermentation tank is detachably provided in the preserving chamber, and the fermentation tank is adapted to slide in the front-rear direction of the fermentation device to be pushed in or out of the preserving chamber.

The fermentation device includes one or more of a stirrer, a hanger, a tray and a fermentation tank. The fermentation device has a plurality of detachable auxiliary accessories, which can adapt to the production requirements of different fermented foods. It is beneficial to the use of various functions of the fermentation device and to the improvement of the user experience of the fermentation device.

Compared with the prior art, the technical solution of the present invention has the following advantages:
In the technical solution of the present invention, the fermentation device includes the fermentation chamber located in the sauce-wine pickling area of the box and the preserving chamber located in the curing area of the box, the preserving chamber being isolated from the fermentation chamber. The preserving chamber and the fermentation chamber, isolated from each other, can provide different fermentation environments for different flavors of fermented foods, so that simultaneous production of different fermented foods is realized. The fermentation device can simultaneously produce at least one type of fermented food at a time, thereby meeting the production needs of home-made fermented foods, effectively improving the utilization rate of different functions of the fermentation device, and effectively improving the user experience of the fermentation device.

### BRIEF DESCRIPTION OF THE DRAWINGS

FIG. 1 is a schematic three-dimensional diagram of an embodiment of a fermentation device of the present invention.
FIG. 2 is a schematic three-dimensional diagram of the interior of a box in the fermentation device shown in FIG. 1.
FIG. 3 is a schematic perspective view of a sauce-wine pickling area in the box in the fermentation device shown in FIG. 1.
FIG. 4 is a schematic three-dimensional diagram of a first housing member in the embodiment of the fermentation device shown in FIG. 3.
FIG. 5 is a schematic three-dimensional diagram of a second housing member in the embodiment of the fermentation device shown in FIG. 3.
FIG. 6 is a schematic three-dimensional diagram of the second housing member provided with a bracket in the embodiment of the fermentation device shown in FIG. 3.
FIG. 7 is a schematic perspective view of a preserving chamber in a curing area of the box in the fermentation device shown in FIG. 1.
FIG. 8 is a schematic three-dimensional diagram of the preserving chamber provided with a hanger and a tray in the embodiment of the fermentation device shown in FIG. 7.
FIG. 9 is a schematic three-dimensional diagram of the preserving chamber provided with a plurality of trays in the embodiment of the fermentation device shown in FIG. 7.
FIG. 10 is a schematic three-dimensional diagram of the preserving chamber provided with a fermentation tank in the embodiment of the fermentation device shown in FIG. 7.

### DETAILED DESCRIPTION

It is known from the related art that existing domestic fermentation devices often have problems of single function and difficulty in meeting consumer demands.

There are many types of fermented foods, mainly including sauce-wine pickled foods and cured foods. The sauce-wine pickled foods include: wine pickled foods such as wine pickled shrimps and wine pickled crabs; sauce pickled foods such as beef sauce and chili sauce; salted foods such as pickled vegetables; pickled foods such as pickles and chicken feet with pickled peppers; and brewed foods such as fermented rice wine and fruit wine. The cured foods include: preserved foods such as preserved meat, sausage and preserved chicken; and air-dried foods such as dried fruits and tea. The production methods, fermentation environments and production cycles of different fermented foods are different.

However, whether it is an independent fermentation device or a fermentation function integrated in other household appliances such as an oven, the domestic fermentation device currently on the market often has only one cavity; in addition, the production time and fermentation environments of different fermented foods are different, and the production of fermented food needs to continue occupying the cavity of the fermentation device. The production of a fermented food will affect the production of other foods. Therefore, existing domestic fermentation device can only produce one type of food at a time, and a user usually only uses a function with short time and high frequency, thereby resulting in the problems of single function and poor consumer experience of the fermentation device.

To resolve the above technical problem, the present invention provides a fermentation device. By providing a fermentation chamber and a preserving chamber, isolated from each other, in the fermentation device, different fermentation environments are provided for different flavors of fermented foods, and simultaneous production of different fermented foods is realized, thereby improving the utilization rate of different functions and improving the user experience.

To make the above objects, features and advantages of the present invention clearer and more comprehensible, specific embodiments of the present invention will be described in detail below with reference to the accompanying drawings.

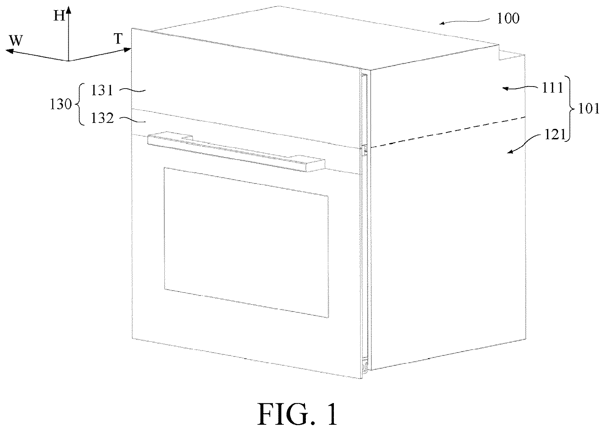

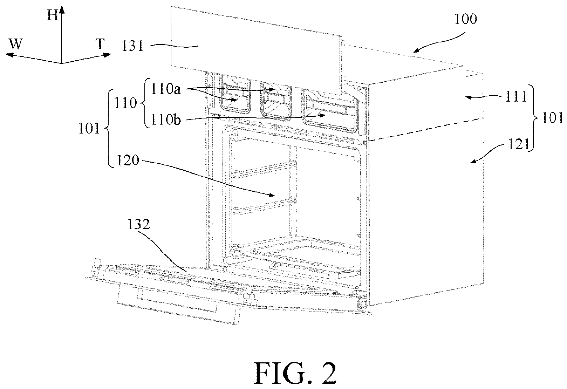

FIG. 1 is a schematic three-dimensional diagram of an embodiment of a fermentation device (100) of the present invention, and FIG. 2 is a schematic three-dimensional diagram of the interior of a box (101) in the fermentation device (100) shown in FIG. 1.

As shown in FIG. 1 and FIG. 2, the fermentation device (100) includes: a box (101), the box (101) including a sauce-wine pickling area (111) and a curing area (121); a fermentation chamber (110), the fermentation chamber (110) being located in the sauce-wine pickling area (111) of the box (101); and a preserving chamber (120), the preserving chamber (120) being located in the curing area (121) of the box (101), and the preserving chamber (120) being isolated from the fermentation chamber (110).

The fermentation device (100) includes a fermentation chamber (110) located in the sauce-wine pickling area (111) of the box (101) and a preserving chamber (120) located in the curing area (121) of the box (101), the preserving chamber (120) being isolated from the fermentation chamber (110). The preserving chamber (120) and the fermentation chamber (110), isolated from each other, can provide different fermentation environments for different flavors of fermented foods, so that simultaneous production of different fermented foods is realized. The fermentation device (100) can simultaneously produce at least one type of fermented food at a time, thereby meeting the production needs of home-made fermented foods, effectively improving the utilization rate of different functions of the fermentation device (100), and effectively improving the user experience of the fermentation device (100).

The box (101) is adapted to house hardware of the fermentation device (100) and provides a fermentation environment for food fermentation. In this embodiment, the box (101) has an opening (not shown). Specifically, the opening is located on a front side of the box (101), that is, in use, the opening is located on a side of the box (101) facing a user.

Referring to FIG. 1 and FIG. 2, in this embodiment, the fermentation device (100) further includes: a door (130) connected to the box (101), the door (130) being adapted to open or close the opening of the box (101), and when the door (130) opens the opening of the box (101), the height of the opening exposed by the door (130) being gradually increased. The method of opening the door (130) of the fermentation device (100) in a height direction (H) can effectively improve the convenience in use of the fermentation device (100), and can also provide greater flexibility for the installation and use of the fermentation device (100).

Especially when the fermentation device (100) is set to be embedded, the door (130) of the fermentation device (100) is opened in a height direction (H) without requiring environments on both sides of the fermentation device (100) in a width direction (W), and the opening of the door (130) is not affected by walls on both sides in the width direction (W), so that the installation flexibility of the embedded fermentation device (100) can be improved.

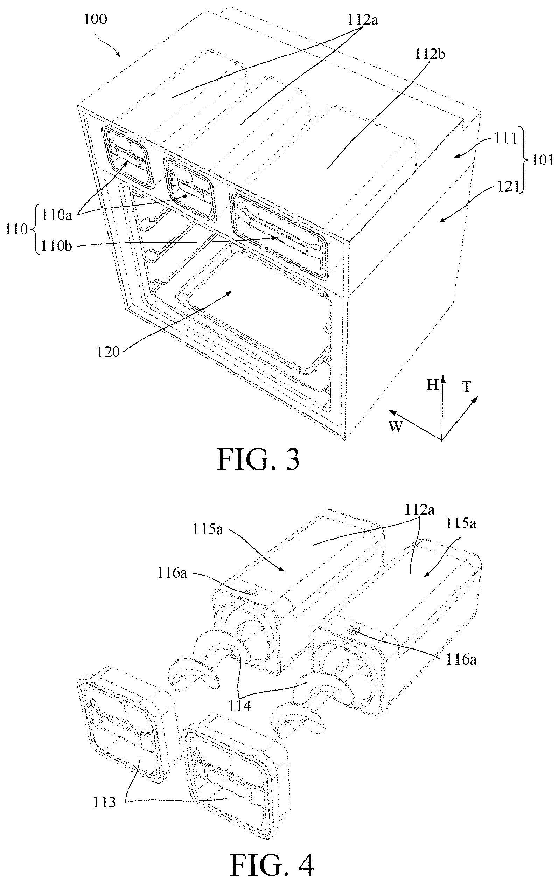

Referring to FIG. 1 to FIG. 3, the fermentation chamber (110) in the sauce-wine pickling area (111) of the box (101) is adapted to house materials and provide a fermentation environment for fermentation. FIG. 3 is a schematic perspective view of the sauce-wine pickling area (111) in the box (101) in the fermentation device (100) shown in FIG. 1.

Fermented foods that can be produced in the sauce-wine pickling area (111) include: wine pickled foods such as wine pickled shrimps and wine pickled crabs; sauce pickled foods such as beef sauce, thick broad-bean sauce and chili sauce; salted foods such as pickled vegetables; pickled foods such as pickles and chicken feet with pickled peppers; and brewed foods such as fermented rice wine and fruit wine.

It should be noted that as shown in FIG. 1 and FIG. 2, in this embodiment, the fermentation device (100) has a plurality of doors (130), the plurality of doors (130) including: a first door (131), the first door (131) being adapted to open or close an opening of the sauce-wine pickling area (111) of the box (101). The opening and closing of the sauce-wine pickling area (111) of the box (101) are controlled by the independent first door (131), so that the isolation of the sauce-wine pickling area (111) can be effectively improved, it is beneficial to simultaneous production of different fermented foods, and it is also beneficial to the improvement of the utilization rate of different functions of the fermentation device (100).

Specifically, the first door (131) is slidably connected to the box (101), and the door (130) slides in the height direction (H) of the fermentation device (100). In this embodiment, the first door (131) slides upward in the height direction (H) of the fermentation device (100) to avoid affecting the use of other functions of the fermentation device (100), thereby improving convenience. In other embodiments of the present invention, the first door (131) may also be disposed to be hingedly connected to the box (101). The manner of connection between the first door (131) and the box (101) is not limited in the present invention.

Referring to FIG. 3 again, in this embodiment, there are a plurality of fermentation chambers (110), and the plurality of fermentation chambers (110) are isolated from each other. Specifically, in this embodiment, three independent fermentation chambers (110) are provided in the sauce-wine pickling area (111) of the box (101). However, in other embodiments of the present invention, the number of fermentation chambers (110) in the sauce-wine pickling area (111) of the box (101) may also be 1, 2, 4, 5, or other numbers. The fermentation device (100) is provided with a plurality of fermentation chambers (110) isolated from each other. Each fermentation chamber (110) can provide an independent fermentation environment. Different materials can be fermented respectively in different fermentation chambers (110), thereby realizing simultaneous production of various fermented foods, and improving the utilization rate of various functions of the fermentation device (100).

Fermented foods such as wine pickled foods, sauce pickled foods, salted foods, pickled foods and brewed foods have a high frequency of daily consumption and a small amount of single-time consumption, and the above fermented foods are not suitable for long-term storage, and they should be eaten immediately after being produced. Therefore, the fermented food produced in the fermentation chamber (110) has a small amount of single-time production and a high production frequency, so that the method of providing a plurality of fermentation chambers (110) in the sauce-wine pickling area (111) of the box (101) can divide the sauce-wine pickling area (111) of the box (101) into a plurality of small spaces, thereby improving the space utilization rate of the sauce-wine pickling area (111) of the box (101) under the premise of satisfying the needs of home-made fermented food production, and improving the utilization rate of different functions of the fermentation device (100).

It should be noted that to realize the formation and control of different fermentation environments and to ensure the quality of fermented foods produced in different fermentation chambers (110), in this embodiment, the fermentation device (100) is also adapted to independently monitor a fermentation time of each fermentation chamber (110), an internal temperature of the fermentation chamber (110), an internal humidity of the fermentation chamber (110), a gas pressure in the fermentation chamber (110), an environment pH value in the fermentation chamber (110), the water activity of an environment in the fermentation chamber (110), and the oxygen content in the fermentation chamber (110), to ensure the stability and control of a fermentation environment in different fermentation chambers (110), thereby ensuring the quality of fermented foods produced by the fermentation device (100). Specifically, the fermentation device (100) further includes control elements such as a temperature module (not shown), a humidity module (not shown), and a gas pressure module (not shown) to monitor the fermentation environment.

Specifically, the fermentation environment in the fermentation chamber (110) can be monitored and controlled by providing an element such as a temperature sensor, a humidity sensor, a pressure sensor, a pH detector, and an oxygen content sensor in each fermentation chamber (110).

The method of providing a plurality of fermentation chambers (110) in the sauce-wine pickling area (111) of the box (101) to divide the sauce-wine pickling area (111) of the box (101) into a plurality of small spaces reduces the volume of a single fermentation chamber (110). Not only the space utilization rate can be improved, but also the difficulty of controlling fermentation environment parameters such as temperature, humidity and pH value in each fermentation chamber (110) can be reduced. It is beneficial to the formation of a stable fermentation environment in each fermentation chamber (110), and to the improvement of the quality of the produced fermented foods.

Referring to FIG. 3 to FIG. 5, the plurality of fermentation chambers (110) include a first fermentation chamber (110a) adapted to perform anaerobic fermentation and a second fermentation chamber (110b) adapted to perform aerobic fermentation or anaerobic fermentation. The fermentation device (100) further includes: a first housing member (112a), the first housing member (112a) being located in the first fermentation chamber (110a), a space defined by an inner surface of the first housing member (112a) being adapted to store materials, and the first housing member (112a) being provided with an inlet valve; and a second housing member (112b), the second housing member (112b) being located in the second fermentation chamber (110b), a space defined by an inner surface of the second housing member (112b) being adapted to store materials, and the second housing member (112b) being provided with an inlet valve and an outlet valve.

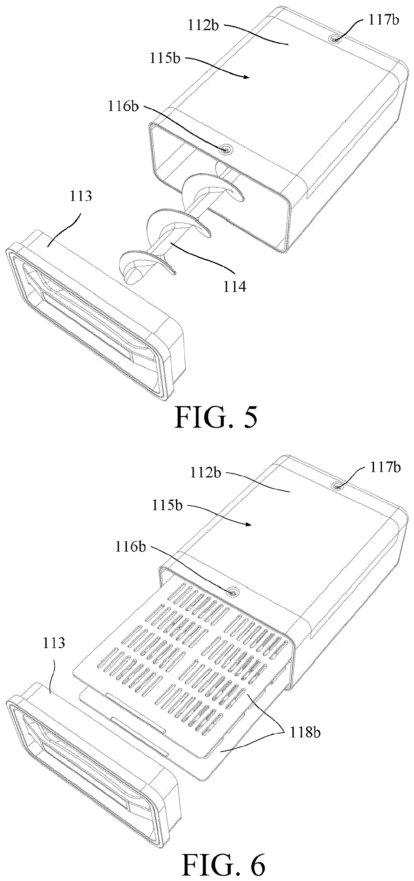

FIG. 4 is a schematic three-dimensional diagram of a first housing member (112a) in the embodiment of the fermentation device (100) shown in FIG. 3. FIG. 5 is a schematic three-dimensional diagram of a second housing member (112b) in the embodiment of the fermentation device (100) shown in FIG. 3.

According to the demands of different fermented foods, fermentation chambers (110) with different fermentation conditions are provided. The function of the fermentation chamber (110) can be effectively expanded, it is beneficial to the improvement of the utilization rate of different fermentation chambers (110), to the improvement of the utilization rate of different functions of the fermentation device (100), and further to the improvement of the user experience of the fermentation device (100).

Specifically, three fermentation chambers (110) including two first fermentation chambers (110a) and one second fermentation chamber (110b) are provided in the sauce-wine pickling area (111) of the box (101). However, in other embodiments of the present invention, the number of fermentation chambers (110) in the sauce-wine pickling area (111) of the box (101) may be set to other numbers, where the number of the first fermentation chambers (110a) and the second fermentation chambers (110b) may also be set to other numbers.

Referring to FIG. 3, with reference to FIG. 4, the first fermentation chamber (110a) is adapted to produce an anaerobic fermented food, so that the first fermentation chamber (110a) is adapted to produce foods fermented by anaerobic bacteria, including: yogurt, wine pickled foods, brewed foods, sauce pickled foods, etc. The first housing member (112a) is adapted to house materials for fermentation.

In this embodiment, the first housing member (112a) can be pulled in a front-rear direction (T) of the fermentation device (100) to be inserted into or pulled out of the first fermentation chamber (110a). The first housing member (112a) is used to house materials to be fermented, so that direct contamination of the first fermentation chamber (110a) by the fermented materials can be effectively avoided, and it is beneficial to the reduction of the difficulty of cleaning during the use of the fermentation device (100). Moreover, the first housing member (112a) is detachably provided in the first fermentation chamber (110a), thereby facilitating the cleaning and use of the first housing member (112a), and facilitating access to produced fermented foods.

The first housing member (112a) is provided with an outlet valve (116a). The outlet valve (116a) is adapted to discharge gas in the first housing member (112a). An anaerobic fermentation environment is formed in the first housing member (112a). Moreover, the outlet valve (116a) is also adapted to release gas generated during the fermentation process to avoid the occurrence of excessive gas pressure. The safety of the fermentation device (100) during use can be effectively improved. The formation and control of a stable fermentation environment can be effectively ensured.

Specifically, as shown in FIG. 4, the first housing member (112a) includes a detachable front cover (113), after the front cover (113) is detached, an opening of the first housing member (112a) is exposed, and filling with a material is realized through the opening of the first housing member (112a).

Referring to FIG. 3, with reference to FIG. 5, the second fermentation chamber (110b) is adapted to produce an anaerobic fermented food and an aerobic fermented food, so that the second fermentation chamber (110b) is adapted to produce foods fermented by anaerobic bacteria, such as yogurt, wine pickled foods, brewed foods and sauce pickled foods, as well as foods fermented by aerobic bacteria, such as strong-smelling tofu, hairy tofu and other fermented soybean products. The second housing member (112b) is adapted to house materials for fermentation.

In this embodiment, the second housing member (112b) can be pulled in the front-rear direction (T) of the fermentation device (100) to be inserted into or pulled out of the second fermentation chamber (110b), so that direct contamination of the second fermentation chamber (110b) by the fermented materials is avoided, to reduce the difficulty of cleaning during the use. Moreover, the second housing member (112b) is detachably provided in the second fermentation chamber (110b) for the purpose of facilitating cleaning and facilitating access to a finished product.

The second housing member (112b) is also provided with an outlet valve (116b) to form an anaerobic fermentation environment and adjust a gas pressure. Moreover, the second housing member (112b) is further provided with an inlet valve (117b). The inlet valve (117b) is adapted to input gas or moisture into the second housing member (112b) to adjust the oxygen content or humidity.

Specifically, the second housing member (112b) also includes a detachable front cover (113), after the front cover (113) is detached, an opening of the second housing member (112b) is exposed, and filling with a material is realized through the opening of the second housing member (112b).

As shown in FIG. 3 to FIG. 5, in this embodiment, the volume of the space defined by the inner surface of the second housing member (112b) is greater than the volume of the space defined by the inner surface of the first housing member (112a). Since the second fermentation chamber (110b) can perform both aerobic fermentation and anaerobic fermentation and the first fermentation chamber (110a) can only perform anaerobic fermentation, second fermentation chamber (110b) has diversified functions. The method of providing a large second fermentation chamber (110b) can effectively improve the utilization rate of the second fermentation chamber (110b).

Moreover, the second fermentation chamber (110b) is also adapted to produce fermented soybean products. The volume of the fermented soybean product is generally relatively large, so that the method of providing a large second housing member (112b) enables the second housing member (112b) in the second fermentation chamber (110b) to produce the fermented soybean product conveniently.

Specifically, the volume of the space defined by the inner surface of the second housing member (112b) is approximately twice the volume of the space defined by the inner surface of the first housing member (112a). However, in other embodiments of the present invention, a ratio of the volume of the space defined by the inner surface of the second housing member (112b) to the volume of the space defined by the inner surface of the first housing member (112a) may be other values.

It should be noted that as shown in FIG. 4 and FIG. 5, in this embodiment, the fermentation device (100) further includes: a stirrer (114), the stirrer (114) being detachably provided in the fermentation chamber (110). In the fermented foods produced by the fermentation device (100), fermented foods such as sauce pickled foods and yogurt are in a fluid or semi-fluid state. The detachable stirrer (114) can stir materials during the process of producing a fluid or semi-fluid fermented food, to improve the uniformity of material fermentation and improve the quality of the formed fermented food. Specifically, each fermentation chamber (110) is provided with a stirring motor, and the stirrer (114) is connected to the stirring motor after being installed in the fermentation chamber (110) to realize stirring of fermented materials.

In addition, referring to FIG. 6, in this embodiment, the fermentation device (100) further includes: a bracket (118b), the bracket (118b) being detachably provided in the second housing member (112b), the bracket (118b) being adapted to slide in a front-rear direction (T) of the fermentation device (100) to be pushed in or out of the second housing member (112b). The bracket (118b) is adapted to hold a solid material, such that various surfaces of the solid material can be sufficiently exposed to a fermentation environment, thereby improving the fermentation effect. FIG. 6 is a schematic three-dimensional diagram of the second housing member (112b) provided with the bracket (118b) in the embodiment of the fermentation device (100) shown in FIG. 3.

In this embodiment, a portion of at least one of the first housing member (112a) and the second housing member (112b) is made of a transparent material. The method of making a portion of the housing member of a transparent material enables real-time observation of the degree of fermentation of materials in the first housing member (112a) and the second housing member (112b), and can better control the degree of fermentation of fermented foods produced in the fermentation chamber (110). It is beneficial to better achievement of various functions of the fermentation device (100) and to the improvement of the user experience.

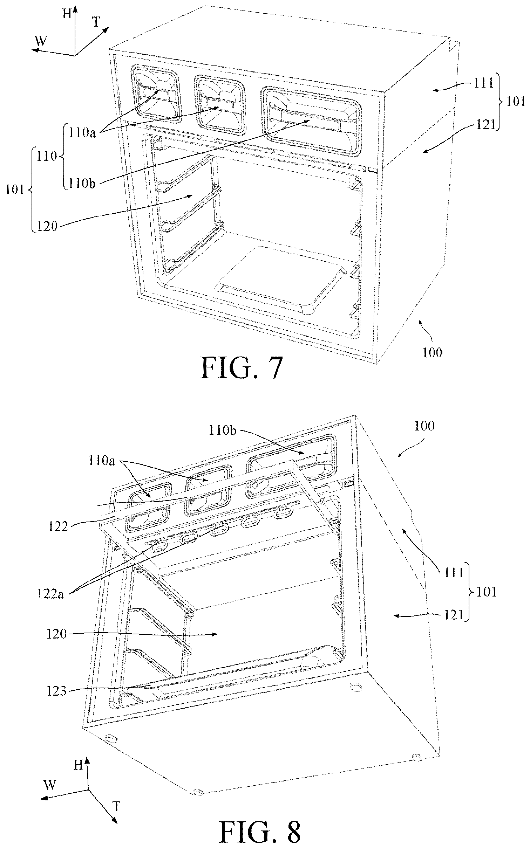

Referring to FIG. 7, the preserving chamber (120) in the curing area (121) of the box (101) is adapted to house materials and provide a fermentation environment to form fermentation. FIG. 7 is a schematic perspective view of the preserving chamber (120) in the curing area (121) of the box (101) in the fermentation device (100) shown in FIG. 1.

Fermented foods that are produced in the curing area (121) shown in FIG. 3 may include: preserved foods such as preserved meat, sausage, preserved duck or preserved fish; air-dried foods such as dried fruits and tea; fermented dough; and brewed foods such as fermented rice wine and fruit wine (grape wine, kiwifruit wine, lotus seed wine, or ginkgo wine).

In this embodiment, the volume of the preserving chamber (120) is greater than the volume of the fermentation chamber (110).

The fermented foods such as preserved foods, air-dried foods and fermented dough are generally large in volume and have a long production cycle, but the above-mentioned fermented foods have low storage requirements and can be stored for a long time. Therefore, the fermented food produced in the preserving chamber (120) has a large amount of single-time production, so that the preserving chamber (120) is large in volume, can meet the volume requirements of different fermented foods under the premise of satisfying the needs of home-made fermented food production, and is beneficial to the improvement of the user experience of the fermentation device (100).

To realize the formation and control of a fermentation environment in the preserving chamber (120) and to ensure the quality of fermented foods produced in the preserving chamber (120), in this embodiment, the fermentation device (100) is also adapted to independently monitor a fermentation time of the preserving chamber (120), an internal temperature of the preserving chamber (120), an internal humidity of the preserving chamber (120), a gas pressure in the preserving chamber (120), an environmental pH value in the preserving chamber (120), the water activity of an environment in the preserving chamber (120), and a ventilation condition in the preserving chamber (120), to ensure the stability and control of a fermentation environment in different preserving chambers (120), thereby ensuring the quality of fermented foods produced by the fermentation device (100). Specifically, the fermentation device (100) further includes control elements such as a temperature module (not shown), a humidity module (not shown), and a gas pressure module (not shown) to monitor the fermentation environment.

Specifically, the fermentation environment in the fermentation chamber (110) can be monitored and controlled by providing an element such as a temperature sensor, a humidity sensor, a pressure sensor, a pH detector, and a fan in the preserving chamber (120).

In this embodiment, the curing area (121) and the sauce-wine pickling area (111) are arranged in a height direction (H) of the fermentation device (100).

The arrangement of the sauce-wine pickling area (111) and the curing area (121) in the height direction (H) can effectively improve the installation flexibility of the fermentation device (100). Especially when the fermentation device (100) is set to be embedded, the method of arranging the sauce-wine pickling area (111) and the curing area (121) in the height direction (H) facilitates the installation and use of embedded device.

Specifically, as shown in FIG. 7, the curing area (121) is located below the sauce-wine pickling area (111).

Since the foods produced in the curing area (121) includes meat fermented foods such as preserved meat, sausage, preserved duck and preserved fish, oil may be separated out from the meat fermented foods during the production process to generate oil droplets. Therefore, the arrangement of the curing area (121) below the sauce-wine pickling area (111) can effectively prevent the oil droplets in the production process of the meat fermented foods from polluting device in the sauce-wine pickling area (111), thereby effectively avoiding unnecessary pollution during use, and effectively reducing the cleaning difficulty of the fermentation device (100).

In addition, referring to FIG. 1, FIG. 2 and FIG. 7, in this embodiment, the fermentation device (100) has a plurality of doors (130), the plurality of doors (130) including: a second door (132), the second door (132) being adapted to open or close an opening of the curing area (121) of the box (101). The opening and closing of the curing area (121) of the box (101) are controlled by the independent second door (132), so that the isolation of the curing area (121) can be effectively improved, it is beneficial to simultaneous production of different fermented foods, and it is also beneficial to the improvement of the utilization rate of different functions of the fermentation device (100).

Specifically, the second door (132) is hingedly connected to the box (101), and a hinging shaft extends in the width direction (W) of the fermentation device (100). The fermentation device (100) is provided with a plurality of doors (130), and the doors (130) are enabled to be opened in the height direction (H) and can be reasonably selected and assembled according to the use requirements. The use convenience can be effectively improved, and it is beneficial to the improvement of the user experience.

In this embodiment, the second door (132) is a pull-down door, that is, a hinging shaft of the second door (132) is located below the box (101). The method of setting the second door (132) as a pull-down door when the curing area (121) is located below the sauce-wine pickling area (111) can prevent the second door (132) from obstructing the observation of the sauce-wine pickling area (111), and the use convenience can be effectively improved.

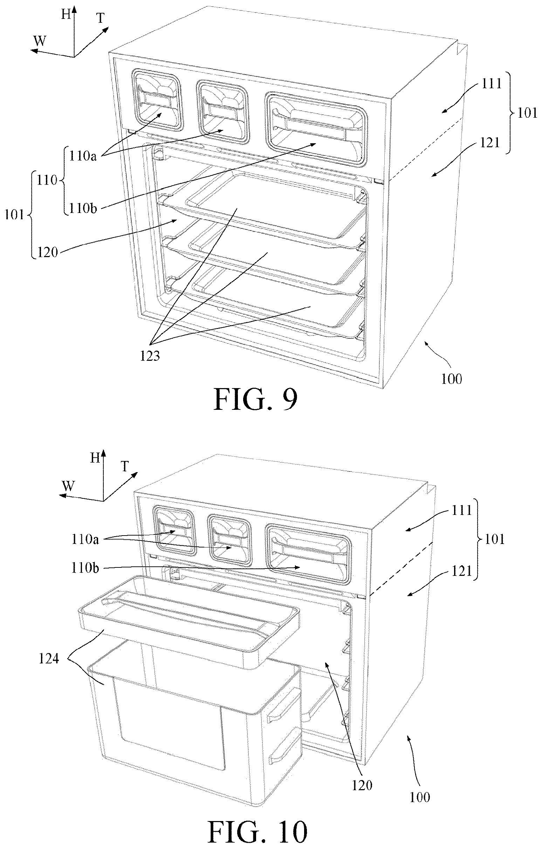

It should be noted that in this embodiment, the fermentation device (100) further includes: a hanger (122) (as shown in FIG. 8), where the hanger (122) is detachably provided in the preserving chamber (120), the hanger (122) is adapted to slide in the front-rear direction (T) of the fermentation device (100) to be pushed in or out of the preserving chamber (120), and the hanger (122) is provided with a hook (122a) slidable in the hanger (122); and one or more trays (123) (as shown in FIG. 8 and FIG. 9), where the tray (123) is detachably provided in the preserving chamber (120), and the tray (123) is adapted to slide in the front-rear direction (T) of the fermentation device (100) to be pushed in or out of the preserving chamber (120). FIG. 8 is a schematic three-dimensional diagram of the preserving chamber (120) provided with the hanger (122) and the tray (123) in the embodiment of the fermentation device (100) shown in FIG. 7. FIG. 9 is a schematic three-dimensional diagram of the preserving chamber (120) provided with a plurality of trays (123) in the embodiment of the fermentation device (100) shown in FIG. 7.

The hanger (122) is adapted to hang materials to improve the fermentation effect. Specifically, the hanger (122) is adapted to hang materials to produce preserved foods. The hook (122a) on the hanger (122) is slidable along the hanger (122) to adjust spacing between the materials hung on the hanger (122). Moreover, as shown in FIG. 8, a tray (123) may be provided below the hanger (122) to collect oil droplets generated during the fermentation of a meat material for convenient cleaning.

The tray (123) is adapted to hold a material. Specifically, the tray (123) is adapted to hold an air-dried food or fermented dough. As shown in FIG. 9, the plurality of trays (123) are stacked in the preserving chamber (120) to improve the space utilization rate of the preserving chamber (120).

In addition, as shown in FIG. 10, in this embodiment, the fermentation device (100) further includes: a fermentation tank (124), where the fermentation tank (124) is detachably provided in the preserving chamber (120), and the fermentation tank (124) is adapted to slide in the front-rear direction (T) of the fermentation device (100) to be pushed in or out of the preserving chamber (120). FIG. 10 is a schematic three-dimensional diagram of the preserving chamber (120) provided with the fermentation tank (124) in the embodiment of the fermentation device (100) shown in FIG. 7.

The fermentation tank (124) is adapted to hold a relatively large volume of fluid or semi-fluid to meet the needs of fermentation of a large volume of fluid or semi-fluid material. It should be noted that in this embodiment, the fermentation tank (124) can be used for making fruit wine (grape wine, kiwifruit wine, lotus seed wine, or ginkgo wine). In addition, since the production of the fruit wine is greatly affected by the season and the amount of single-time production is large, the fermentation chamber (110) can also be used for making the fruit wine, thereby meeting the demands for making a large amount of fruit wine.

In this embodiment, the fermentation device (100) has a plurality of detachable auxiliary accessories, which can adapt to the production requirements of different fermented foods. It is beneficial to the use of various functions of the fermentation device (100) and to the improvement of the user experience of the fermentation device (100).

It should be noted that in this embodiment, the fermentation device (100) has the stirrer (114), the hanger (122), the tray (123) and the fermentation tank (124). In other embodiments of the present invention, the fermentation device may include only one or more of a stirrer, a hanger, a tray and a fermentation tank.

Although the present invention has been disclosed above, the present invention is not limited thereto. Any person skilled in the art can make various variations and modifications to the present invention without departing from the spirit and scope of the present invention. Therefore, the protection scope of the present invention shall be subject to the scope of the claims.

## Claims

1. A fermentation device (100), comprising:
a box (101), the box (101) comprising a sauce-wine pickling area (111) and a curing area (121);
a fermentation chamber (110), the fermentation chamber (110) being located in the sauce-wine pickling area (111) of the box (101); and
a preserving chamber (120), the preserving chamber (120) being located in the curing area (121) of the box (101), and the preserving chamber (120) being isolated from the fermentation chamber (110).

2. The fermentation device (100) according to claim 1, **characterized in that** the curing area (121) and the sauce-wine pickling area (111) are arranged in a height direction (H) of the fermentation device (100).

3. The fermentation device (100) according to claim 2, **characterized in that** the curing area (121) is located below the sauce-wine pickling area (111).

4. The fermentation device (100) according to claim 1 or 2, **characterized in that** the box (101) has an opening, wherein
the fermentation device (100) further comprises: a door (130) connected to the box (101), the door (130) being adapted to open or close the opening of the box (101), and when the door (130) opens the opening of the box (101), the height of the opening exposed by the door (130) being gradually increased.

5. The fermentation device (100) according to claim 4, **characterized in that** the door (130) is slidably connected to the box (101), and the door (130) slides in the height direction (H) of the fermentation device (100).

6. The fermentation device (100) according to claim 4, **characterized in that** the door (130) is hingedly connected to the box (101), and a hinging shaft extends in a width direction (W) of the fermentation device (100).

7. The fermentation device (100) according to claim 4, **characterized in that** the fermentation device (100) comprises a plurality of doors (130), the plurality of doors (130) comprising: a first door (131), the first door (131) being adapted to open or close an opening of the sauce-wine pickling area (111) of the box (101); and a second door (132), the second door (132) being adapted to open or close an opening of the curing area (121) of the box (101).

8. The fermentation device (100) according to claim 1, **characterized in that** there is a plurality of fermentation chambers (110), and the plurality of fermentation chambers (110) is isolated from each other.

9. The fermentation device (100) according to claim 8, **characterized in that** the plurality of fermentation chambers (110) comprises a first fermentation chamber (110a) adapted to perform anaerobic fermentation and a second fermentation chamber (110b) adapted to perform aerobic fermentation or anaerobic fermentation, wherein
the fermentation device (100) further comprises: a first housing member (112a), the first housing member (112a) being located in the first fermentation chamber (110a), a space defined by an inner surface of the first housing member (112a) being adapted to store materials, and the first housing member (112a) being provided with an outlet valve (116a); and
a second housing member (112b), the second housing member (112b) being located in the second fermentation chamber (110b), a space defined by an inner surface of the second housing member (112b) being adapted to store materials, and the second housing member (112b) being provided with an inlet valve (117b) and an outlet valve (116b).

10. The fermentation device (100) according to claim 9, **characterized in that** the volume of the space defined by the inner surface of the second housing member (112b) is greater than the volume of the space defined by the inner surface of the first housing member (112a).

11. The fermentation device (100) according to claim 9, **characterized by** further comprising: a bracket (118b), the bracket (118b) being detachably provided in the second housing member (112b), the bracket (118b) being adapted to slide in a front-rear direction (T) of the fermentation device (100) to be pushed in or out of the second housing member (112b).

12. The fermentation device (100) according to claim 1, **characterized by** further comprising: one or more of a stirrer (114), a hanger (122), a tray (123), and a fermentation tank (124), wherein
the stirrer (114) is detachably provided in the fermentation chamber (110);
the hanger (122) is detachably provided in the preserving chamber (120), the hanger (122) is adapted to slide in the front-rear direction (T) of the fermentation device (100) to be pushed in or out of the preserving chamber (120), and the hanger (122) is provided with a hook (122a) slidable in the hanger (122);
the tray (123) is detachably provided in the preserving chamber (120), and the tray (123) is adapted to slide in the front-rear direction (T) of the fermentation device (100) to be pushed in or out of the preserving chamber (120); and
the fermentation tank (124) is detachably provided in the preserving chamber (120), and the fermentation tank (124) is adapted to slide in the front-rear direction (T) of the fermentation device (100) to be pushed in or out of the preserving chamber (120).
